## Europäisches Patentamt

## European Patent Office

## Office européen des brevets

(19)

(11) Veröffentlichungsnummer: **0 297 618**
**A2**

(12) **EUROPÄISCHE PATENTANMELDUNG**

(21) Anmeldenummer: **88110591.0**

(51) Int. Cl.4: **C12N 15/00 , A01H 1/00**

(22) Anmeldetag: **02.07.88**

---

| | |
|---|---|
| Patentansprüche für folgenden Vertragsstaat: ES. | (71) Anmelder: **HOECHST AKTIENGESELLSCHAFT**<br>**Postfach 80 03 20**<br>**D-6230 Frankfurt am Main 80(DE)** |
| (30) Priorität: **02.07.87 DE 3721840**<br>          **30.09.87 DE 3732972** | (72) Erfinder: **Strauch, Eckhard, Dr.**<br>**Rosenheide 2**<br>**D-4800 Bielefeld(DE)** |
| (43) Veröffentlichungstag der Anmeldung:<br>**04.01.89 Patentblatt 89/01** | Erfinder: **Wohlleben, Wolfgang, Dr.**<br>**Menzelstrasse 1**<br>**D-4800 Bielefeld(DE)** |
| (84) Benannte Vertragsstaaten:<br>**AT BE CH DE ES FR GB GR IT LI LU NL SE** | Erfinder: **Pühler, Alfred, Prof. Dr.**<br>**Am Waldschlösschen 2**<br>**D-4800 Bielefeld(DE)** |

---

(54) **Resistenzgen gegen Phosphinothricin und seine Verwendung.**

(57) Durch Selektion von <u>Streptomyces</u> <u>viridochromo-</u><u>genes</u> DSM 4112 gegen Phosphinothricyl alanyl ala-nin (PTT) erhält man PTT-resistente Selektanten. Aus der Gesamt-DNA dieser Selektanten erhält man durch Schneiden mit <u>Nco</u>I, Klonieren eines 2,7 kb großen Fragments und Selektion auf PTT-Resistenz das DNA-Fragment, welches das Phosphinothricin (PTC)-Resistenzgen trägt. Dieses ist zur Herstellung PTC-resistenter Pflanzen, aber auch als Resistenz-Marker geeignet.

FIG.1

Xerox Copy Centre

EP 0 297 618 A2

## Resistenzgen gegen Phosphinothricin und seine Verwendung

Phosphinothricin (PTC, 2-Amino-4-methylphosphinobuttersäure) ist ein Glutaminsynthetase-Inhibitor. PTC ist ein "Baustein" des Antibiotikums Phosphinothricyl-alanylalanin. Dieses Tripeptid (PTT) ist aktiv gegen Gram-positive und Gram-negative Bakterien und auch gegen den Pilz Botrytis cinerea (Bayer et al., Helv. Chim. Acta 55 (1972) 224). PTT wird von dem Stamm Streptomyces viridochromogenes Tü 494 produziert. Dieser Stamm ist bei der Deutschen Sammlung von Mikroorganismen, Mascheroder Weg l b, D-3300 Braunschweig, in der allgemeinen Sammlung hinterlegt und unter der Nummer DSM 40736 allgemein zugänglich. Unter den Bedingungen des Budapester Vertrags wurde derselbe Stamm am 13.5.1987 erneut hinterlegt; er ist unter der Nummer DSM 4112 allgemein zugänglich (und wird im folgenden durch diese Nummer identifiziert).

Aus der Deutschen Patentschrift 2 717 440 ist es bekannt, daß PTC als Totalherbizid wirkt. In der veröffentlichten PCT-Anmeldung WO 86/02097 sind Pflanzen beschrieben, deren Resistenz gegen PTC darauf zurückzuführen ist, daß sie Glutaminsynthetase überproduzieren. Solche Überproduktionen, beispielsweise infolge einer Genamplifikation, bergen jedoch die Gefahr der Instabilität in sich. Im Falle einer solchen Instabilität ginge also die Überproduktion an Glutaminsynthetase zurück und die kompetitive Inhibitorwirkung des PTC käme wieder zum Zuge.

In der (nicht veröffentlichten) Europäischen Patentanmeldung mit der Veröffentlichungsnummer (EP-A) 0 257 542 wurde bereits ein Resistenzgen gegen PTC vorgeschlagen, das erhältlich ist aus der Gesamt-DNA von auf PTT-Resistenz selektiertem Streptomyces viridochromogenes DSM 4112 durch Schneiden mit BamHI, Klonieren eines 4,0 kb großen Fragments und Selektion auf PTT-Resistenz.

Es wurde nun gefunden, daß bei der Selektion auf PTT-resistente Kolonien von S. viridochromogenes DSM 4112 nach längerer Inkubation (≧ 1 Woche) zwei Phänotypen unterschieden werden können:

Typ I verhindert in seiner direkten Umgebung in jedem Fall das Hintergrundwachstum sensitiver Kolonien auf PTT-haltigem Medium. Aus der Gesamt-DNA dieser Selektanten läßt sich das in der EP-A 0 257 542 vorgeschlagene 4,0 kb BamHI-Fragment isolieren. Um Typ II können dagegen nicht resistente Bakterien in einem Bereich von etwa 5 mm Durchmesser auf Selektionsmedium (Minimalmedium mit PTT) langsam wachsen. Durch Einzelkolonieausstrich können die Selektanten vom Typ II rein isoliert werden. Diese Selektanten enthalten das erfindungsgemäße Resistenzgen gegen PTT. Hieraus ergibt sich, in Analogie zur EP-A 0 257 542, daß dieses Gen eine Resistenz gegen PTC vermittelt, so daß im folgenden auch der Begriff "PTC-Resistenz" gebraucht wird. Die Erfindung betrifft außerdem die Verwendung dieses Gens zur Herstellung PTC-resistenter Pflanzen sowie als Marker und zwar als PTT-Resistenzmarker in Bakterien und als PTC-Resistenzmarker in Pflanzenzellen.

Die Erfindung betrifft weiterhin Pflanzenzellen und Pflanzen, deren Samen und Teile, die das erfindungsgemäße Resistenzgen enthalten.

Das Antibiotikum PTT wird von Bakterien aufgenommen und zu PTC abgebaut. Dieses inhibiert bei Bakterien ebenfalls die Glutaminsynthetase, so daß die Bakterien an Glutaminmangel sterben. PTT-produzierende Bakterien sollten daher einen Mechanismus besitzen, der sie vor der Wirkung des PTT schützt, also entweder die Wiederaufnahme des produzierten PTT verhindert oder eine Modifikation des Abbauproduktes PTC ermöglicht. Überraschenderweise ist der PTT-Produzent S. viridochromogenes DSM 4112 aber gegen sein eigenes Antibiotikum sensitiv. Es gelang aber, durch Selektion auf PITT-Resistenz unter etwa $10^5$ Selektanten diejenigen zu finden, die gegen PTT resistent sind darunter die erfindungsgemäßen Selektanten vom Typ II.

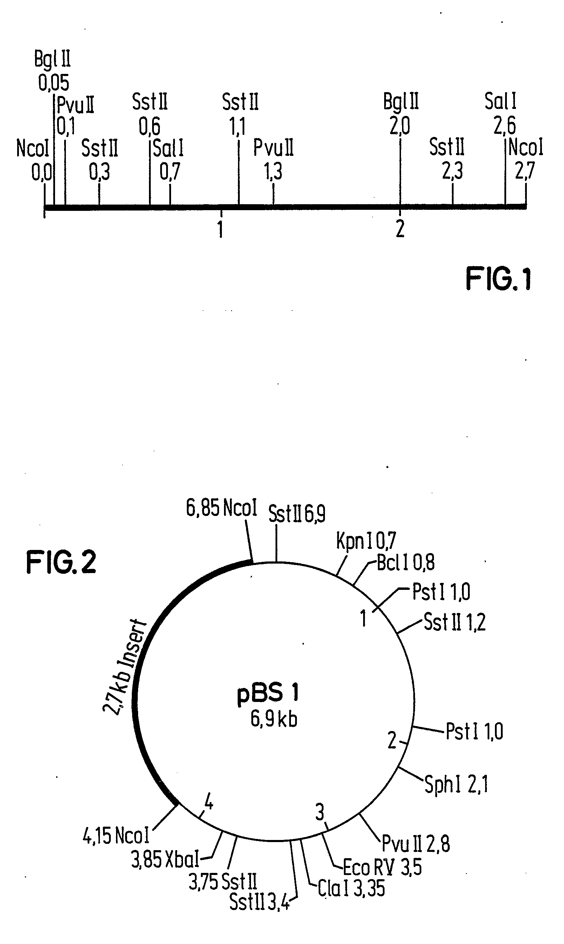

Aus der DNA dieser Selektanten wurde eine Genbank angelegt, indem die DNA isoliert, mit NcoI gespalten und in einen Streptomycetenvektor ligiert wurde. Das Ligationsgemisch wurde in den handelsüblichen Stamm S. lividans TK 23 transformiert, wobei je 1 μg Ligationsgemisch etwa 5000 bis 10000 Transformanten mit einem Insert von etwa 1 bis 5 kb erhalten wurden. Unter den Transformanten finden sich PTT-resistente S. lividans-Stämme. Durch Isolierung des Plasmids und Retransformation in S. lividans konnte gezeigt werden, daß die Resistenz plasmidcodiert ist. Das für die Resistenz verantwortliche Gen liegt auf einem 2,7 kb-NcoI-Fragment (Figur 1). Dieses Fragment enthält keine Schnittstellen für die Enzyme BamHI, BclI, ClaI, EcoRI, EcoRV, HindIII, HpaI, KpnI, PstI, SmaI, SphI, SstI und XhoI. Aus den Selektanten vom Typ II kann außerdem - wie in der EP-A 0 257 542 vorgeschlagen - das dort definierte PTC-Resistenzgen isoliert werden. Umgekehrt konnte unter diesen Bedingungen aus den Selektanten vom Typ I das erfindungsgemäße Resistenzgen nicht isoliert werden.

Der Vergleich mit der Restriktionskarte eines nicht näher charakterisierten Resistenzgens aus S. hygroscopicus FERM BP-130/ATCC 21705 (EP-A 0 173 327, Figur 7; vgl. auch die nicht vorveröffentlichten EP-A 0 242 236 und 0 242 246 bzw. WO 87/05 629, die dasselbe Gen betreffen) zeigt, daß das erfindungsgemäße Resistenzgen von dem bekannten Gen verschieden ist, welches auf der Suche nach den PTT-Biosynthesegenen gefunden wurde. Dasselbe gilt für das in der EP-A 0 257 542 vorgeschlagene Resistenzgen.

In den folgenden Beispielen wird die Erfindung näher erläutert. Teile und Prozentangaben beziehen sich auf das Gewicht, sofern keine anderen Angaben gemacht werden.

Beispiel 1: PTT-resistente Selektanten

Der Stamm S. viridochromogenes DSM 4112 wurde auf MinimalMedium (Hopwood et al., Genetic Manipulation of Streptomyces, A Laboratory Manual, The John Innes Foundation, Norwich, England (1985), S. 233) angezogen und mit PTT in steigenden Konzentrationen versetzt. Bei einer Konzentration von 50 µg/ml wurde pro etwa $10^5$ Kolonien zwei resistente Kolonien gefunden. Bei längerer Inkubation (7 Tage, 30° C) zeigten sich auf den PTT-haltigen Minimalmediumplatten zwei unterschiedliche Kolonietypen. Typ I inhibiert in seiner direkten Umgebung verstärkt das Hintergrundwachstum sensitiver Kolonien. Aus der Gesamt-DNA dieser Mutanten läßt sich das in EP-A 0 257 542 vorgeschlagene 4,0 kb BamHI-Fragment isolieren.

Daneben findet sich auf den Selektionsplatten noch ein zweiter Resistenztyp (Typ II). In der direkten Umgebung dieser Mutanten können sensitive Kolonien auf PTT-haltigem Medium wachsen. Resistente Mutanten sind vom Wildtyp, der schon bei Konzentrationen von 5 µg/ml inhibiert wird, deutlich und reproduzierbar zu unterscheiden.

Beispiel 2: Isolierung des Resistenzgens

Aus den Selektanten vom Typ II gemäß Beispiel 1 isoliert man die Gesamt-DNA und spaltet sie mit NcoI. Das Plasmid pGM4 (EP-A 0 257 417, Beispiel 1, Figur 4) wird ebenfalls mit NcoI geöffnet und mit Alkalischer Phosphatase behandelt. Anschließend werden die beiden Ansätze vereinigt und ligiert. Das Ligationsgemisch wird nach S. lividans TK 23 (erhältlich bei der John Innes Foundation) transformiert, wobei je 1 µg Ligationsgemisch 5000 bis 10000 Transformanten mit einem Insert von etw = 1 - 5 kb erhalten werden. Selektion auf PITT-Resistenz ergibt resistente S. lividans-Kolonien mit dem Phänotyp II. Aus diesen wird das aufgenommene Plasmid isoliert und mit NcoI geschnitten. Man findet ein 2,7 kb NcoI-Fragment, welches das für die Resistenz verantwortliche Gen trägt. Dieses Plasmid erhielt die Bezeichnung pBSI (Figur 2). Es ist 6,9 kb groß, d.h. das 0,55 kb NcoI-Fragment aus pGM4 ist durch das 2,7 kb Insert mit dem Resistenzgen ersetzt. Dieses Insert ist in beiden Orientierungen eingebaut und in jeder Orientierung aktiv.

Durch Retransformation in S. lividans TK 23 kann gezeigt werden, daß die PTT-Resistenz plasmidcodiert ist, da die Transformanten auf Minimalmedium wachsen, das 30 µg/ml PTT enthält.

Ansprüche

1. Resistenzgen gegen Phosphinothricin (PTC), lokalisiert auf einem DNA-fragment, das erhältlich ist aus der Gesamt-DNA von auf Phosphinothricylalanyl-alanin (PTT)-Resistenz selektiertem Streptomyces viridochromogenes DSM 4112 durch Schneiden mit NcoI, Klonieren eines 2,7 kb großen Fragments und Selektion auf PTT-Resistenz.

2. Resistenzgen gegen PTC, lokalisiert auf einem 2,7 kb DNA-Fragment, das gekennzeichnet ist durch die Restriktionskarte gemäß Figur 1.

3. Verwendung des Gens nach Anspruch 1 oder 2 zur Herstellung PTC-resistenter Pflanzen.

4. Verwendung des Gens nach Anspruch 1 oder 2 als PTT-Resistenz-marker in Bakterien.

5. Verwendung des Gens nach Anspruch 1 oder 2 als PTC-Resistenzmarker in Pflanzenzellen.

6. Pflanzenzellen, Pflanzen, deren Samen und Teile, gekennzeichnet durch ein Gen nach Anspruch 1 oder 2.

Patentansprüche für folgenden Vertragsstat: ES

1. Verfahren zur Gewinnung eines Resistenzgens gegen Phosphinothricin (PTC), dadurch gekennzeichnet, daß man aus der Gesamt-DNA von auf Phosphinothricyl-alanyl-alanin (PTT)-Resistenz selektiertem Streptomyces viridochromogenes DSM 4112 durch Schneiden mit NcoI ein 2,7 kb großes Fragment isoliert, dieses ganz oder ein das Resistenzgen enthaltendes Teilfragment davon kloniert und auf PTT-Resistenz selektiert.

2. Verwendung des nach Anspruch 1 erhältlichen Gens zur Herstellung PTC-resistenter Pflanzen.

3. Verwendung des nach Anspruch 1 erhältlichen Gens als PTT-Resistenzmarker in Bakterien.

4. Verwendung des nach Anspruch 1 erhältlichen Gens als PTC-Resistenzmarker in Pflanzenzellen.

FIG.1

FIG.2